# EUROPEAN PATENT SPECIFICATION

(11) **EP 0 499 718 B1**
(45) Date of publication and mention of the grant of the patent: **08.05.1996**
(21) Application number: 91122356.8
(22) Date of filing: 30.12.1991
(51) Int. Cl.: A61M 1/28

(54) **Set of tubes intended for peritoneal dialysis**
Schlauchsatz für die peritoneale Dialyse
Ensemble de tubes pour la dialyse péritonéale

(30) Priority: 18.02.1991 SE 9100471
(43) Date of publication of application: 26.08.1992
(73) Proprietor: GAMBRO AB, S-220 10 Lund (SE)
(72) Inventor: Jeppsson, Jan-Bertil, S-234 00 Lomma (SE); Losell, Ingvar, S-245 00 Staffanstorp (SE)
(74) Representative: Asketorp, Göran

(56) References cited:
- EP-A- 0 028 371
- DE-A- 2 535 191
- US-A- 4 950 259

## Description

### FIELD OF INVENTION

The present invention relates to a set of tubes intended for peritoneal dialysis comprising a patient tube having a first end which is provided with a connector assembly for fluid communication with the patient's peritoneal cavity, and a second end for fluid communication with a supply tube for supply of fresh dialysis solution and a discharge tube for discharge of spent dialysis solution.

### PRIOR ART

Peritoneal dialysis is a well-known and, for a long time, proven alternative to haemodialysis for cleansing of the blood of patients who have failing kidneys or whose kidneys do not function at all.

Examples of various systems and apparatus intended for peritoneal dialysis can be found in, for example, EP-A-0 335 814, WO-A-84/02277, GB-A-2 009 619, US-A-Re 32 303 and US-A-4 252 115. In the described systems are used peritoneal catheters of the type which are described in, for example, WO-A-86/06282 and US-A-4 935 004.

The connection of the different systems to the peritoneal catheter can be carried out with the help of connectors, for example, of the type described in US-A-4 636 204.

A longer or shorter patient tube forms a part of all systems, which tube attaches the patient's peritoneal catheter to the rest of the system. For the patient's comfort, this patient tube is often given a length of 2-3 meters. In this way, a considerable return of already used or spent dialysis solution results at each change of solution.

US-A-4 950 259 discloses a double lumen peritoneal catheter including an outer tube and an inner tube fully contained within the lumen of the outer tube and able to move freely within the outer lumen. An inflow connector tube is attached to the inner tube and an outflow connector tube is attached to the outer tube. The outer tube is perforated to allow inflow of peritoneal fluid. This dual lumen catheter permits continous flushing, which should inhibit fibrin accumuluation reducing the risk of blockage of the perforations and tubal lumens.

### DESCRIPTION OF THE INVENTION

The present invention is intended to solve the above mentioned problems and this is achieved in that the patient tube is provided with a double lumen construction along at least a part of its length with a first channel for the fresh dialysis solution and a second channel for the spent dialysis solution, and in that the patient tube comprises a chamber connected to both the first channel and the second channel and positioned adjacent the first end of the patient tube closest to the patient.

At its second end, away from the patient, the patient tube normally comprises an inlet connector or similar for fluid communication with the supply tube and the discharge tube. According to the invention, this inlet connector is provided with an inlet means for fluid communication between said supply tube and said first channel and an outlet means for fluid communication between said discharge tube and said second channel. Alternatively, the same operation can be achieved with the help of a suitable valve construction.

The first end of the patient tube, nearest the patient, normally comprises a connector assembly piece provided with said chamber, a connector arrangement for connection to a patient's peritoneal catheter, and a single lumen patient conduit interconnecting the connector piece and the connector arrangement. Preferably, the connector piece comprises a first portion for receiving the first end of the patient tube; a connection nipple for fluid communication with the patient conduit; and an open chamber for fluid communication with both the first channel and second channel and the connection nipple.

In a preferred embodiment of the set of tubes according to the invention, the supply tube has a first end for fluid communication with said first channel and a second end for fluid communication with a bag or similar intended for control of the weight and/or the volume of supplied dialysis fluid, and/or for fluid communication with a source of fresh dialysis solution. Moreover, the discharge tube has a first end for fluid communication with said second channel and a second end for fluid communication with a bag or similar intended for control of the weight and/or the volume of spent dialysis fluid, and/or for fluid communication with a bag or similar intended for collection of waste.

A simple and unexpensive design is achieved, if the patient tube is divided in two symmetrical semicircular channels by a partition wall. At the same time there is also obtained an efficient heat transfer between the spent dialysis solution and the fresh solution.

A further improved heat transfer is achieved, if the patient tube is divided into four symmetrical channels by two perpendicular partition walls. Two diagonally opposite channels can by such a design be used for the fresh dialysis solution and the other two for the spent solution. A further advantage by such a design is that the tube is not so easy to bend. Consequently, the risk for blocking of any of the channels by such a bending or knicking is reduced.

### BRIEF DESCRIPTION OF THE DRAWINGS

Fig. 1 is a schematic sideview of a preferred embodiment of the set of tubes according to the invention.

Fig. 2 is a cross-sectional view of an inlet connector included in the set of tubes.

Fig. 3 is an endview of the inlet connector according to fig. 2 seen from beneath.

Fig. 4 is a sideview of a connector piece included in the set of tubes.

Fig. 5 is a schematical sectional view through a patient tube according to the invention divided into two semicircular halves or channels.

Fig. 6 is a cross-sectional view similar to Fig. 5 through a preferred embodiment of the patient tube according to the invention divided into four channels.

Fig. 7, finally, is a cross-sectional view similar to Fig. 5 through an alternative embodiment of the patient tube according to the invention also divided into four channels.

### BEST MODE OF CARRYING OUT THE INVENTION

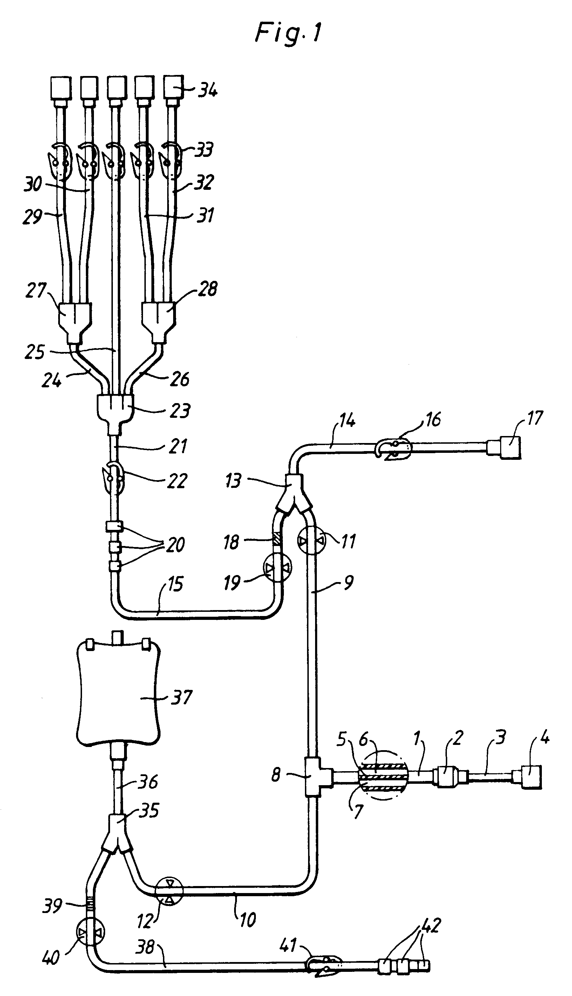

Fig. 1 shows a preferred embodiment of the set of tubes according to the invention. Concerning how the shown components can be constructed in more detail, reference is made to that which is generally known, for example from the above mentioned patent publications.

The shown set of tubes consists of a patient tube 1 which, via a connector piece 2, communicates with a patient conduit 3 which, in turn, is connectable with the help of a connector arrangement 4 to a patient's peritoneal catheter.

The patient tube 1 is divided into two channels by means of common partition wall 5, that is, one channel 6 for fresh dialysis solution and one channel 7 for the spent dialysis solution. At its second end, away from the patient, the patient tube 1 is connected via a T-connector 8 to both a tube 9 for the supply of fresh dialysis solution and to a tube 10 for the discharge of spent dialysis solution. The tube 9 can be closed with the help of a clamp arrangement 11 and, in the same way, the tube 10 can be closed with the help of a clamp arrangement 12. The tube 9 communicates with two tubes 14 and 15 via a Y-piece 13. The tube 14 is provided with a tube clamp 16 and is terminated with a connector arrangement 17 intended to be connected to a bag or similar for control of the weight and/or the volume of supplied dialysis fluid.

The tube 15 is provided with a coloured, for example green, indication 18 which is intended to simplify the attachment of the complete set of tubes to a dialysis monitor. From the indication, the tube 15 extends through a clamp arrangement 19 and is terminated with a connector arrangement 20 which connects this to a tube 21 which is provided with a tube clamp 22. The tube 21 is terminated with a connector piece 23 which connects this to three tubes 24, 25 and 26. The tubes 24 and 26 are terminated in turn with the help of Y-pieces 27 and 28, which communicate with tubes 29, 30, 31 and 32. All tubes 29, 30, 25, 31 and 32 are provided with tube clamps 33 and are terminated with connector arrangements 34. These connector arrangements 34 are intended to be connected to different sources of fresh dialysis solution, for example five bags containing said solution. Supply can hereby be achieved via the force of gravity by placing the bags at a suitable height, or with the help of suitable pump arrangements. In the shown example, five connector arrangements 34 are shown. In practice any from one to ten can be used.

The tube 10 is terminated with a Y-piece 35 which connects this to a tube 36 and a bag 37. The bag 37 is intended to be used for control of the weight and/or the volume of spent dialysis fluid. The bag can for example be placed on scales. The tube 10 is, via the Y-piece 35, also connected to a discharged tube 38 which is provided with, for example, a blue colour marking 39, which has the same function as the colour marking 18. The tube 38 extends from the colour marking 39 via a clamp arrangement 40 and a tube clamp 41 to a connector arrangement 42 which is intended to connect the tube to a bag or similar intended for collection of waste.

The T-connector 8 shown in figs. 1-3 can alternatively be formed as, for example, a Y-piece. What is hereby important is only that the inlets 9' and 10' which are intended to be connected to the tubes 9, 10 respectively, are separated from each other via a partition wall 5'. The semi-circular shaped channels 6' and 7' are both separated from each other and communicate with just one of the inlets 9', 10' respectively. Figure reference numeral 43 denotes a ring-shaped channel in which the patient tube 1 is intended to be inserted. At the same time, the partition wall 5 is inserted into a slot 44. Reference numeral 45 denotes recesses from mould cores which are necessary for the forming of the channels 6' and 7'.

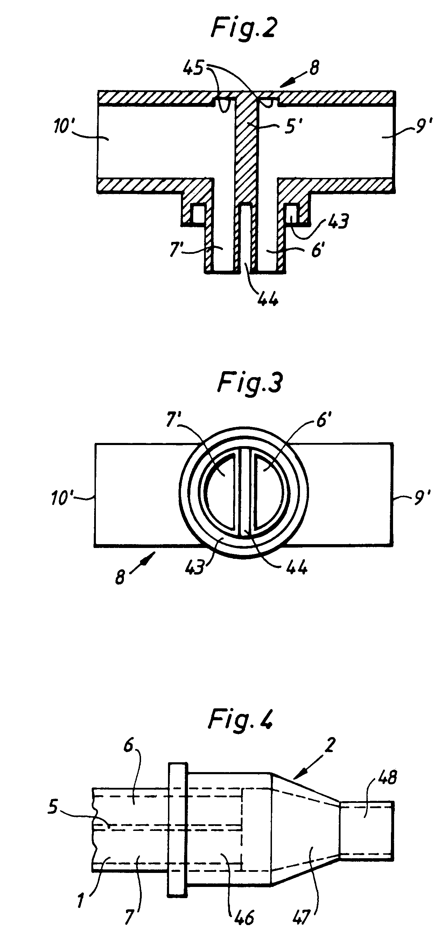

In fig. 4 there is shown an example of how the connector piece 2 can be formed. A section of the patient tube 1 is hereby shown inserted in a cavity 46 which leads into an open chamber 47. The two channels 6 and 7 are thus placed in open communication with this chamber 47. Via a connection nipple 48, they thereafter communicate with the patient conduit 3 shown in fig. 1.

Thanks to the invention, only the quantity of the spent dialysis solution which remains between the patient and the connector piece 2 is returned to the patient. Moreover, fresh dialysis solution is supplied via the channel 6 and this is pre-heated, via the partition wall 5, by the spent dialysis solution which is discharged via the channel 7.

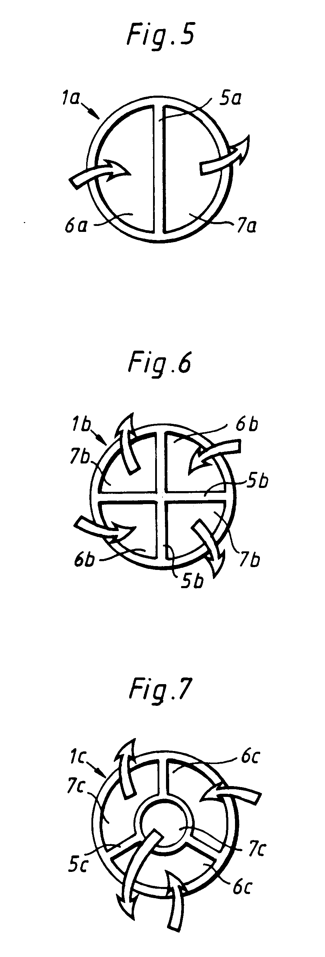

Fig 5-7, finally, show sections through three different patient tubes made in accordance with the present invention. The three tubes have been given the reference numerals 1a, 1b and 1c, respectively. The tube 1a is divided into two channels 6a and 7a by a partition wall 5a. In a similar way the tube 1b is divided into four channels 6b, 6b, 7b and 7b by two perpendicularly arranged partition walls 5b. The tube 1c, finally, is divided into four channels 6c, 6c, 7c and 7c by a modified arrangement of partition walls 5c. The two tubes 1b and 1c have the advantage over the tube 1a in that they are more difficult to bend. Furthermore, the heat transfer between the spent dialysis solution and the fresh solution is improved.

Naturally, the invention is not restricted to solely the above described embodiments, but can be varied within the scope of the following claims. By way of example, many components can be modified in accordance with that which is described in the above mentioned patent publications. Alternatively, the two channels 6 and 7 can be formed as two concentrically arranged circular shaped channels, i.e. with an inner tube arranged centrally in an outer tube. In addition, the shown set of tubes can be complemented with further components if so required, for example with a pump unit if it is desired to pump the various liquids.

## Claims

1. Set of tubes intended for peritoneal dialysis comprising a patient tube (1) having a first end which is provided with a connector assembly (2,3,4) for fluid communication with the patient's peritoneal cavity, and a second end for fluid communication with a supply tube (9) for supply of fresh dialysis solution and a discharge tube (10) for discharge of spent dialysis solution, **characterized** in that the patient tube (1) is of double lumen construction along at least a part of its length with a first channel (6) for the fresh dialysis solution and a second channel (7) for the spent dialysis solution, and in that the patient tube (1) comprises a chamber (2,3,47) adjacent the first end connected to both the first channel (6) and the second channel (7).

2. Set of tubes according to claim 1, whereby the patient tube (1) comprises an inlet connector (8) or similar at the second end for fluid communication with the supply tube (9) and the discharge tube (10), **characterized** in that the inlet connector (8) is provided with an inlet means (6',9') for fluid communication between said supply tube (9) and said first channel (6) and an outlet means (7',10') for fluid communication between said discharge tube (10) and said second channel (7).

3. Set of tubes according to claim 2, **characterized** in that said inlet connector (8) comprises a first portion for connection to said second end of the double lumen patient tube (1), said first portion having arrangements (43,44) of a complementary shape to said second end; a second portion (9') for connection with the supply tube; a third portion (10') for connection with the discharge tube; and partition means (5') for preventing direct fluid communication between said second portion (9') and said third portion (10').

4. Set of tubes according to claim 1, 2 or 3, **characterized** in that said connector assembly (2,3,4) comprises a connector piece (2) provided with said chamber (47), a connector arrangement (4) for connection to a patient's peritoneal catheter, and a single lumen patient conduit (3) interconnecting said connector piece (2) and said connector arrangement (4).

5. Set of tubes according to claim 4, **characterized** in that said connector piece (2) comprises a first portion (46) for receiving the first end of the patient tube (1); a connection nipple (48) for fluid communication with the patient conduit (3); an open chamber (47) for fluid communication with both said first channel (6) and second channel (7) and said connection nipple (48).

6. Set of tubes according to any one of the previous claims, **characterized** in that the supply tube (9) has a first end for fluid communication with said first channel (6) and a second end for fluid communication (17) with a bag or similar intended for control of the weight and/or the volume of supplied dialysis fluid, and/or for fluid communication with a source of fresh dialysis solution (34).

7. Set of tubes according to any one of the previous claims, **characterized** in that the discharge tube (10) has a first end for fluid communication with said second channel (7) and a second end for fluid communication with a bag (37) or similar intended for control of the weight and/or the volume of spent dialysis fluid (35,36), and/or for fluid communication with a bag or similar intended for collection of waste (42).

8. Set of tubes according to any of the previous claims, **characterized** in that the patient tube (1b or 1c) has more than two channels, preferably four (6b,6b,7b,7b or 6c,6c,7c,7c).

9. Set of tubes according to claim 8, **characterized** in that two (6b,6b or 6c,6c) of the channels are intended for fresh dialysis solution for the supply thereof and in that two other channels (7b,7b or 7c,7c) are intended for spent dialysis solution for the discharge thereor.

10. Set of tubes according to any of the claims 1-7, **characterized** in that the patient tube (1) is divided into two symmetrical semicircular channels by a partition wall (5).

11. Set of tubes according to claims 8 or 9, **characterized** in that the patient tube (1b) is divided into four symmetrical channels by two perpendicular partition walls (5b).

## Patentansprüche

1. Schlauchsatz für die peritoneale Dialyse mit einem Patientenschlauch (1) mit einem ersten Ende, welches mit einem Verbinderaufbau (2, 3, 4) für die Fluidverbindung mit der peritonealen Kavität des Patienten versehen ist, und einem zweiten Ende für die Fluidverbindung mit einem Zuführschlauch (9) für die Zuführung frischer Dialyselösung und einem Abflußschlauch (10) für den Abfluß von verbrauchter Dialyselösung, **dadurch gekennzeichnet,** daß der Patientenschlauch (1) längs mindestens eines Teils seiner Länge einen Doppellumenaufbau aufweist mit einem ersten Kanal (6) für die frische Dialyselösung und einem zweiten Kanal (7) für die verbrauchte Dialyselösung und daß der Patientenschlauch (1) eine Kammer (2, 3, 47) neben dem ersten Ende aufweist, welche sowohl mit dem ersten Kanal (6) als auch mit dem zweiten Kanal (7) verbunden ist.

2. Schlauchsatz nach Anspruch 1, wobei der Patientenschlauch (1) einen Einlaßverbinder (8) oder dergleichen an dem zweiten Ende für die Fluidverbindung mit dem Zuführschlauch (9) und dem Abflußschlauch (10) aufweist, **dadurch gekennzeichnet,** daß der Einlaßverbinder (8) mit einen Einlaßmittel (6', 9') für die Fluidverbindung zwischen dem Zuführschlauch (9) und dem ersten Kanal (6) und einer Auslaßeinrichtung (7', 10) für die Fluidverbindung zwischen dem Abflußschlauch (10) und dem zweiten Kanal (7) versehen ist.

3. Schlauchsatz nach Anspruch 2, **dadurch gekennzeichnet,** daß der Einlaßverbinder (8) einen ersten Teil aufweist für die Verbindung mit dem zweiten Ende des Patientenschlauches (1) mit Doppellumen, wobei der erste Teil Anordnungen (43, 44) einer zu dem zweiten Ende komplementären Gestalt hat; einen zweiten Teil (9') für die Verbindung mit dem Zuführschlauch; einen dritten Teil (10') für die Verbindung mit dem Abflußschlauch; und Trennmittel (5') zur Verhinderung der direkten Fluidverbindung zwischen dem zweiten Teil (9') und dem dritten Teil (10').

4. Schlauchsatz nach Anspruch 1, 2 oder 3, **dadurch gekennzeichnet,** daß der Verbinderaufbau (2, 3, 4) ein Verbinderstück (2), welches mit der Kammer (47) versehen ist, eine Verbinderanordnung (4) für die Verbindung mit einem peritonealen Katheder eines Patienten und eine Patientenleitung (3) mit einem einzigen Lumen aufweist, welche das Verbinderstück (2) mit der Verbinderanordnung (4) verbindet.

5. Schlauchsatz nach Anspruch 4, **dadurch gekennzeichnet,** daß das Verbinderstück (2) einen ersten Teil (46) zur Aufnahme des ersten Endes des Patientenschlauches (1); einen Verbindungsnippel (48) für die Fluidverbindung mit der Patientenleitung (3); und eine offene Kammer (47) aufweist für die Fluidverbindung sowohl mit dem ersten Kanal (6) als auch dem zweiten Kanal (7) und dem Verbindungsnippel (48).

6. Schlauchsatz nach einem der vorhergehenden Ansprüche, **dadurch gekennzeichnet,** daß der Zuführschlauch (9) ein erstes Ende hat für die Fluidverbindung mit dem ersten Kanal (6) und ein zweites Ende hat für die Fluidverbindung (17) mit einem Beutel oder dergleichen, der für die Steuerung des Gewichtes und/oder des Volumens des zugeführten Dialysefluids gedacht ist, und/oder für die Fluidverbindung mit einer Quelle für frische Dialyselösung (34).

7. Schlauchsatz nach einem der vorhergehenden Ansprüche, **dadurch gekennzeichnet,** daß der Abflußschlauch (10) ein erstes Ende hat für die Fluidverbindung mit dem zweiten Kanal (7) und ein zweites Ende hat für die Fluidverbindung mit einem Beutel (37) oder dergleichen, der dafür gedacht ist, das Gewicht und/oder das Volumen des verbrauchten Dialysefluids (35, 36) zu steuern, und/oder für die Fluidverbindung mit einem Beutel oder dergleichen, der für das Sammeln von Abfall (42) gedacht ist.

8. Schlauchsatz nach einem der vorhergehenden Ansprüche, **dadurch gekennzeichnet,** daß der Patientenschlauch (1b oder 1c) mehr als zwei Kanäle hat, vorzugsweise vier (6b, 6b, 7b, 7b oder 6c, 6c, 7c, 7c).

9. Schlauchsatz nach Anspruch 8, **dadurch gekennzeichnet,** daß zwei (6b, 6b oder 6c, 6c) der Kanäle für frische Dialyselösung für ihre Zuführung gedacht sind und daß zwei andere Kanäle (7b, 7b, oder 7c, 7c) für verbrauchte Dialyselösung für ihr Abfließen gedacht sind.

10. Schlauchsatz nach einem der Ansprüche 1 bis 7, **dadurch gekennzeichnet,** daß der Patientenschlauch (1) durch eine Trennwand (5) in zwei symmetrische, halbkreisförmige Kanäle aufgeteilt ist.

11. Schlauchsatz nach Anspruch 8 oder 9, **dadurch gekennzeichnet,** daß der Patientenschlauch (1b) durch zwei senkrechte Trennwände (5b) in vier symmetrische Kanäle aufgeteilt ist.

## Revendications

1. Ensemble de tubes pour la dialyse péritonéale, comprenant un tube de patient (1) ayant une première extrémité équipée d'un ensemble de connexion (2, 3, 4) pour assurer le passage des liquides dans la cavité péritonéale du patient, et une deuxième extrémité pour assurer le passage du liquide dans un tube d'alimentation (9) pour fournir de la solution de dialyse fraîche et dans un tube d'évacuation (10) pour l'évacuation de la solution de dialyse épuisée, caractérisé en ce que le tube de patient (1) a une disposition à double passage sur au moins une partie de sa longueur, avec un premier canal (6) pour la solution de dialyse fraîche et un deuxième canal (7) pour la solution de dialyse épuisée, et en ce que le tube de patient (1) comprend une chambre (2, 3, 47) proche de la première extrémité connectée au premier canal (6) et au deuxième canal (7).

2. Ensemble de tubes selon la revendication 1, dans lequel le tube de patient (1) comprend un connecteur d'entrée (8) ou un dispositif semblable à la deuxième extrémité, pour assurer le passage des liquides dans le tube d'alimentation (9) et dans le tube d'évacuation (10), caractérisé en ce que le connecteur d'entrée (8) est pourvu d'un moyen d'entrée (6', 9') pour assurer le passage du liquide entre ledit tube d'alimentation (9) et ledit premier canal (6), et un moyen de sortie (7', 10') pour le passage du liquide entre ledit tube d'évacuation (10) et le deuxième canal (7).

3. Ensemble de tubes selon la revendication 2, caractérisé en ce que ledit connecteur d'entrée (8) comprend une première partie pour la connexion à ladite deuxième extrémité du tube de patient à double passage (1), ladite première partie ayant des dispositifs (43, 44) de forme complémentaire à ladite deuxième extrémité; une deuxième partie (9') pour la connexion au tube d'alimentation; une troisième partie (10') pour la connexion au tube d'évacuation; et un moyen de séparation (5') pour empêcher le passage direct du liquide entre ladite deuxième partie (9') et ladite troisième partie (10').

4. Ensemble de tubes selon la revendication 1, 2 ou 3, caractérisé en ce que ledit ensemble de connexion (2, 3, 4) comprend une pièce de connecteur (2) disposée dans ladite chambre (47), un dispositif de connexion (4) pour assurer la connexion au cathéter péritonéal d'un patient et une conduite de patient (3) à un seul passage interconnectant ladite pièce de connecteur (2) et ledit dispositif de connexion (4).

5. Ensemble de tubes selon la revendication 4, caractérisé en ce que ladite pièce de connexion (2) comprend une première partie (46) destinée à recevoir la première extrémité du tube de patient (1), un raccord de connexion (48) pour assurer le passage du liquide dans la conduite de patient (3), une chambre ouverte (47) pour assurer le passage du liquide dans ledit premier canal(6) et le deuxième canal (7) et ledit raccord de connexion (48).

6. Ensemble de tubes selon l'une quelconque des revendications précédentes, caractérisé en ce que le tube d'alimentation (9) a une première extrémité pour assurer le passage du liquide dans ledit premier canal (6) et une deuxième extrémité pour assurer le passage du liquide(17) dans un sac ou un dispositif semblable destiné à régler le poids et/ou le volume de liquide de dialyse fourni et/ou pour assurer le passage du liquide provenant d'une source de solution de dialyse fraîche (34).

7. Ensemble de tubes selon l'une quelconque des revendications précédentes, caractérisé en ce que le tube d'évacuation (10) a une première extrémité pour assurer le passage du liquide dans ledit deuxième canal (7), et une deuxième extrémité pour assurer le passage du liquide dans un sac (37) ou un dispositif semblable, destiné à régler le poids et/ou le volume de liquide de dialyse épuisé (35, 36) et/ou pour assurer le passage du liquide dans un sac ou un dispositif semblable destiné à recueillir les déchets (42).

8. Ensemble de tubes selon l'une quelconque des revendications précédentes, caractérisé en ce que le tube de patient (1b ou 1c) présente plus de deux canaux et, de préférence, 4 canaux (6b, 6b, 7b, 7b ou 6c, 6c, 7c, 7c).

9. Ensemble de tubes selon la revendication 8, caractérisé en ce que deux des canaux (6b, 6b ou 6c, 6c) sont destinés à la solution de dialyse fraîche pour fournir celle-ci, et en ce que deux autres canaux (7b, 7b ou 7c, 7c) sont destinés à la solution de dialyse épuisée pour évacuer celle-ci.

10. Ensemble de tubes selon l'une quelconque des revendications 1 à 7, caractérisé en ce que le tube de patient (1) est divisé en deux canaux semi-circulaires symétriques par une paroi séparatrice (5).

11. Ensemble de tubes selon la revendication 8 ou 9, caractérisé en ce que le tube de patient (1b) est divisé en quatre canaux symétriques par deux parois séparatrices perpendiculaires (5b).
